# EUROPEAN PATENT SPECIFICATION

(11) **EP 0 362 926 B1**
(45) Date of publication and mention of the grant of the patent: **26.05.1993**
(21) Application number: 89202397.9
(22) Date of filing: 25.09.1989
(51) Int. Cl.: A61K 35/78, A61K 9/14, A23L 1/308

(54) **Psyllium-containing products**
Psyllium enthaltende Produkte
Produits contenant du psyllium

(30) Priority: 03.10.1988 US 252848
(43) Date of publication of application: 11.04.1990
(73) Proprietor: THE PROCTER & GAMBLE COMPANY, Cincinnati, Ohio 45202 (US)
(72) Inventor: Leis, Paul Dale, Jr., Hamilton, OH 45011 (US); Burns, Larry Earl, Goshen, OH 45122 (US); Hafer, Karen Rose, Cincinnati, OH 45240 (US)
(74) Representative: Suslic, Lydia

(56) References cited:
- US-A- 3 148 114
- US-A- 4 737 364
- US-A- 4 766 004

## Description

The present invention relates psyllium husk of a certain small particle size distribution; and to products containing such particle size psyllium husk suitable for oral administration, especially products to be mixed in liquids to form psyllium-containing drinks.

Products containing psyllium seed husk are known (e.g., Metamucil®, sold by The Procter & Gamble Company). Such products are useful for the benefits of normalizing bowel function and laxation. In addition, recent research has demonstrated the effectiveness of psyllium seed husk fiber in reducing human serum cholesterol levels, and in controlling blood glucose levels in diabetics. See, for example, J. W. Anderson, et al., Fed. Proc., 46, 877 (1987); J. W. Anderson, et al., Am. J. Gastroenterol., 81, 907-919 (1986); and S. Faberberg, Curr. Ther. Res., 31, 166 (1982).

The palatability of psyllium-containing products vary depending on the form used, and, of course, the user's particular preference. Frequently, however, psyllium-containing products are viewed as having poor palatability. In particular, psyllium-containing products to be mixed with a liquid to form a drink are considered by many to be aesthetically objectionable for one or more of several reasons such as texture (e.g., grittiness, general mouth feel), viscosity and visual appearance.

Therefore, improving the palatability of psyllium-containing products is a continuing need which would benefit a significant number of consumers. More palatable products may result in improved compliance for dosing regimens involving several doses or extended duration therapy. Thus, while psyllium can be (and in fact has been) combined with many carriers and flavorants in many forms, there continues to be a need for improved, highly palatable psyllium-containing products.

Current psyllium-containing products, such as products to be mixed with a liquid to form a drink, utilize substantially more of larger particle size psyllium husk. It has been discovered by the present invention that by selecting a particular range of substantially smaller particle size psyllium, the aesthetics of psyllium-containing products are improved dramatically. In addition, in view of a large body of literature that states or intimates that reducing the particle size of other fiber materials (such as wheat bran) reduces the fiber's efficacy [e.g., See: Dietary Fiber in Health and Disease, pages 10-11 (Vahouny and Kritchevsky, editors; Plenum Press, New York, New York; 1982); CRC Handbook of Dietary Fiber in Human Nutrition, pages 267-269 (Spiller, editor; CRC Press, Inc.; Boca Raton, Florida; 1986); Handbook of Dietary Fiber, An Applied Approach, pages 152-153 (Dreher; Marcel Dekker, Inc., New York, New York; 1987); Kirwan et al., British Medical Journal, 4, pages 187-189 (1974); Brodribb et al., Gut, 19, pages 60-63 (1978); and Cummings et al., CMA Journal, 123, pages 1109-1114 (1980)], the present invention is even more surprising since the psyllium of the present invention is essentially at least as efficacious as the substantially larger particle size psyllium used previously.

It is therefore an object of the present invention to provide psyllium husk and psyllium-containing products for oral administration having improved aesthetics. It is a further object to provide psyllium husk and psyllium-containing products to be mixed with a liquid to form a drink which are efficacious and have improved aesthetics, including texture, mouth feel, palatability, grittiness, and/or visual appearance.

These and other objects of the present invention will become readily apparent from the detailed description which follows.

All percentages and ratios used herein are by weight unless otherwise specified.

### SUMMARY OF THE INVENTION

The present invention relates to psyllium husk consisting of particle sizes distributed as follows: less than 15% larger than 177 microns (80 mesh), at least 45% within the range of from 177 microns (80 mesh) to 74 microns (200 mesh), and less than 40% smaller than 74 microns (200 mesh).

The present invention further relates to psyllium-containing products for oral administration comprising: (a) psyllium husk having particle size distributions according to the present invention; and (b) carrier material suitable for oral administration to a human. Preferred are psyllium-containing products containing psyllium having at least 95% purity. Also preferred are psyllium-containing products in a form suitable for mixing with a liquid to form a drink, especially such products wherein the psyllium is agglomerated.

### DETAILED DESCRIPTION OF THE INVENTION

### 1. Psyllium Husk of Limited Small Particle Size:

The present invention relates to certain small particle size psyllium husk from psyllium seed, from plants of the Plantago genus. Various species such as Plantago lanceolate, P. rugelii, and P. major are known. Commercial psyllium husk include the French (black; Plantago indica), Spanish (P. psyllium) and Indian (blonde; P. ovata). Indian (blonde) psyllium husk is preferred for use herein. Also preferred is psyllium husk which is at least 95% pure, more preferably more than 95% pure, and most preferably at least 97% pure.

Psyllium husk of the present invention comprises particle sizes distributed as follows: less than 15% larger than 177 microns (80 mesh), at least 45% within the range of from 177 microns (80 mesh) to 74 microns (200 mesh), and less than 40% smaller than 74 microns (200 mesh). Preferred are particle size distribution of: less than 10% larger than 177 microns (80 mesh), at least 65% within the range of from 177 microns (80 mesh) to 74 microns (200 mesh), and less than 25% smaller than 74 microns (200 mesh). More preferred are particle size distribution of: less than 5% larger than 177 microns (80 mesh), at least 75% within the range of from 177 microns (80 mesh) to 74 microns (200 mesh), and less than 20% smaller than 74 microns (200 mesh).

It is also preferred that the psyllium husk further comprise less than 5% of particle sizes larger than 250 microns (60 mesh), and most preferably essentially no particle sizes larger than 250 microns (60 mesh). Also preferred is less than 5% larger than 177 microns (80 mesh), and most preferred is essentially no particle sizes larger than 177 microns (80 mesh) and less than 25% larger than 149 microns (100 mesh). Particle sizes and particle size distributions may be readily determined by one of ordinary skill in the art, for example by sieving using an Alpine Laboratory Air Jet Sieve, Type 200 LS (sold by Alpine American Corp., Natick Mass.).

The psyllium husk is obtained from the seed coat of psyllium seed. It is typical to remove the seed coat from the rest of the seed by, for example, slight mechanical pressure, and then to use only the seed coat. The seed coat is preferably removed and sanitized by methods known in the art (e.g., ethylene oxide) prior to reducing the particle size to that described herein. Methods for reducing psyllium particle size to those of the present invention are known in the art, or preferably may be reduced by the use of a stud mill (also known as "pin mills") under conditions which selectively reduce the psyllium husk size relative to non-husk impurity, thereby allowing for further purification if so desired.

### 2. Psyllium-containing Products:

The present invention also relates to psyllium-containing products. These products comprise psyllium husk having particle sizes distributed according to the present invention, and one or more carrier materials suitable for oral administration to a human. Preferably, these products comprise from 1% to 99% psyllium husk, and from 1% to 99% carrier material; and more preferably from 10% to about 98% psyllium husk and from 2% to 90% carrier material.

The carrier materials useful for the products of the present invention must be safe for oral administration to humans, and may be chosen by one of ordinary skill in the art as appropriate for the form and use intended for the product. Psyllium-containing product forms, methods for making, and carrier materials useful for these products, are described more fully, for example, in U.S. Patent 4,459,280, to Colliopoulos et al., issued July 10, 1984; U.S. Patent 4,548,806, to Colliopoulos et al., issued October 22, 1985; and U.S. Patent 4,321,263, to Powell et al., issued March 23, 1982.

Most preferred are products of the present invention in dry powder form suitable for mixing in a liquid (typically water) to form a psyllium-containing drink. Preferred carrier materials for such powder forms are known and are described in detail, for example, in U.S. Patents 4,459,280 and 4,548,860. Preferred are such powders (preferably sugar free) comprising maltodextrin. Also especially preferred are powders comprising agglomerates of psyllium and/or coated psyllium, especially agglomerated with maltodextrin and/or sucrose.

Psyllium-containing powders suitable for mixing in a liquid comprising from 10% to 98% of psyllium husk having particle sizes distributed according to the present invention (more preferably from 20% to 95% psyllium husk), and from 0% to 60% maltodextrin (more preferably from 2% to 50% maltodextrin) are preferred. Psyllium-containing products according to the present invention containing sugar (e.g., sucrose) comprise from 10% to 60% (preferably from 20% to 55%) psyllium husk, from 35% to 90% sugar, and from 0% to 5% maltodextrin. Sugar free products typically comprise from 40% to 98% (preferably from 50% to 95%) of psyllium husk according to the present invention, and from 1% to 60% (preferably from 2% to 50%) of maltodextrin. Preferred compositions comprise agglomerated psyllium, and also preferred are compositions wherein the carrier material comprises citric acid.

Ingestion of from 1 gram to 30 grams per day of the psyllium husk according to the present invention is appropriate in most circumstances to produce laxation. However, this can vary with the size and condition of the patient, and such matters will, of course, be up to the attending physician. However, since the psyllium material is non-toxic, even higher ingestion levels can be used without undue side effects. A typical dose for laxation purposes involves administering from 2.5 to 15 grams of psyllium husk in one dose.

The following examples further describe and demonstrate embodiments within the scope of the present invention. These examples are given solely for the purpose of illustration and are not to be construed as limitations of the present invention, as many variations thereof are possible without departing from the spirit and scope.

### EXAMPLE 1

Raw blond psyllium husk [95% purity; ethylene oxide sanitized; particle size distribution approximately: on 20 mesh (841 microns) = 4.6%, thru 20 on 40 mesh (420 microns) = 62.6%, thru 40 on 60 mesh (250 microns) = 23.1%, thru 60 on 100 mesh (149 microns) = 7.2%, thru 100 on 140 mesh (105 microns) = 1.4%, thru 140 on 200 mesh (74 microns) = 0.7%, thru 200 mesh (< 74 microns) = 0.4%] is selectively comminuted by using an Alpine Pin Mill (Model No. 160 UPZ; flow rate = 70 kg/hr; full pin density; 18,000 rpms; sold by Alpine American Corp., Natick, Mass.) to maximize the through 177 microns (80 mesh) on 74 microns (200 mesh) fraction. The comminuted psyllium husk obtained has approximately the following particle size distribution: on 80 mesh (> 177 microns) = 15%; thru 80 on 100 mesh (177-149 microns) = 2.5%; thru 100 on 120 mesh (149-125 microns) = 14.2%; thru 120 on 140 mesh (125-105 microns) = 14.9%; thru 140 on 170 mesh (105-95 microns) = 17.7%; thru 170 on 200 mesh (95-74 microns) = 16.0%; thru 200 on 325 mesh (74-44 microns) = 11.2%; and thru 325 mesh (< 44 microns) = 8.5%.

The selectively comminuted psyllium husk is purified by sifting over a 250 microns (60 mesh) screen to sieve out much of the dark material, and then sieved to collect thru 177 microns (80) on 74 microns (200 mesh) particle size psyllium husk according to the present invention. This psyllium is then used to prepare the following sugared product prepared by dry mixing the ingredients in a Hobart mixer (Model No. N-50).

| Ingredient | Percent of Formula |
|---|---|
| Sucrose | 63.43 |
| Citric Acid | 2.54 |
| Orange Flavor | 1.59 |
| Psyllium | 32.35 |
| FD&C Yellow No. 6 | 0.10 |

One tablespoon of this product is dispersed in 0,23 ℓ (8 oz.) of water to produce a drink containing approximately 5.1 grams of psyllium. Consumption of this drink by a human is effective for providing laxative benefits.

### EXAMPLE 2

Utilizing psyllium husk milled as described in Example 1 collected thru 177 microns 80 mesh, the following sugar free product is prepared.

| Ingredient | Percent of Formula |
|---|---|
| Psyllium | 75.80 |
| Maltrin | 5.50 |
| Citric Acid | 11.50 |
| Potassium Citrate | 1.14 |
| Aspartame | 1.16 |
| Flavor | 4.80 |
| FD&C Yellow No. 5 | 0.067 |
| FD&C Yellow No. 6 | 0.048 |

The composition is prepared by dry mixing the ingredients less approximately 3% of the maltrin, and then agglomerating with the remainder of the maltrin (aqueous solution) in a top spray, fluid bed agglomerater. One teaspoon of this product is dispersed in 0.23 ℓ (8 ounces) of water to product a drink containing approximately 3.4 grams of psyllium. Consumption of this drink by a human is effective for providing laxative benefits.

## Claims

1. Psyllium husk consisting of particle sizes distributed as follows : less than 15% larger than 177 microns (80 mesh), at least 45% within the range of from 177 to 74 microns (80 mesh to 200 mesh), and less than 40% smaller than 74 microns (200 mesh).

2. Psyllium husk according to Claim 1 consisting of particle sizes distributed as follows : less than 5% larger than 250 microns (60 mesh), less than 10% larger than 177 microns (80 mesh), at least 65% within the range of from 177 to 74 microns (80 mesh to 200 mesh), and less than 25% smaller than 74 microns (200 mesh).

3. Psyllium husk according to Claim 2 consisting of the following particle size distribution : no particle sizes larger than 60 mesh, less than 5% larger than 177 microns (80 mesh), at least 75% within the range of from 177 microns to 74 microns (80 mesh to 200 mesh), and less than 20% smaller than 74 microns (200 mesh).

4. Psyllium husk according to Claim 3 consisting of the particle size distribution of : no particles larger than 177 microns (80 mesh), at least 80% within the range of from 177 to 74 microns (80 mesh to 200 mesh), and less than 20% smaller than 74 microns (200 mesh).

5. Psyllium-containing products for oral administration comprising:
(a) psyllium husk having particle size distribution according to any of Claims 1-4; and
(b) carrier materials suitable for oral administration to a human.

6. Psyllium-containing products according to Claim 5 in a powder form suitable for mixing with a liquid to form a psyllium-containing drink.

7. Psyllium-containing products according to Claims 5 or 6 further comprising agglomerates of psyllium.

8. Psyllium-containing products according to any of Claims 5-7 wherein said carrier materials comprise materials selected from sucrose, maltodextrin, citric acid, and combinations thereof.

9. Psyllium-containing products in a powder form suitable for mixing in a liquid according to any of Claims 5-8 consisting of:
(a) from 10% to 60% psyllium husk;
(b) from 35% to 90% sugar; and
(c) from 0% to 5% maltodextrin.

10. Psyllium-containing products in a powder form suitable for mixing in a liquid according to any of Claims 5-8 consisting of:
(a) from 40% to 98% psyllium husk; and
(b) from 1% to 60% maltodextrin.

## Patentansprüche

1. Psylliumschalen, die aus Teilchengrößen bestehen, welche wie folgt verteilt sind: weniger als 15 % größer als 177 µm (US-Siebnummer 80), wenigstens 45 % in einem Bereich von 177 µm bis 74 µm (US-Siebnummern 80 bis 200), und weniger als 40 % kleiner als 74 µm (US-Siebnummer 200).

2. Psylliumschalen nach Anspruch 1, die aus Teilchengrößen bestehen, welche wie folgt verteilt sind: weniger als 5 % größer als 250 µm (US-Siebnummer 60), weniger als 10 % größer als 177 µm (US-Siebnummer 80), wenigstens 65 % in einem Bereich von 177 µm bis 74 µm (US-Siebnummern 80 bis 200), und weniger als 25 % kleiner als 74 µm (US-Siebnummer 200).

3. Psylliumschalen nach Anspruch 2, welche die folgende Teilchengrößenverteilung aufweisen: keine größeren Teilchengrößen als jene, die der US-Siebnummer 60 entsprechen, weniger als 5 % größer als 177 µm (US-Siebnummer 80), wenigstens 75 % in einem Bereich von 177 µm bis 74 µm (US-Siebnummern 80 bis 200), und weniger als 20 % kleiner als 74 µm (US-Siebnummer 200).

4. Psylliumschalen nach Anspruch 3, welche die folgende Teilchengrößenverteilung aufweisen: keine Teilchen größer als 177 µm (US-Siebnummer 80), wenigstens 80 % in einem Bereich von 177 bis 74 µm (US-Siebnummern 80 bis 200), und weniger als 20 % kleiner als 74 µm (US-Siebnummer 200).

5. Psyllium enthaltende Produkte für die orale Verabreichung, umfassend:
(a) Psylliumschalen mit einer Teilchengrößenverteilung nach einem der Ansprüche 1 bis 4; und
(b) Trägermaterialien, welche für die orale Verabreichung an einen Menschen geeignet sind.

6. Psyllium enthaltende Produkte nach Anspruch 5, in einer Pulverform, welche zum Mischen mit einer Flüssigkeit unter Bildung eines Psyllium enthaltenden Getränkes geeignet ist.

7. Psyllium enthaltende Produkte nach Anspruch 5 oder 6, welche weiterhin Psylliumagglomerate enthalten.

8. Psyllium enthaltende Produkte nach einem der Ansprüche 5 bis 7, wobei die genannten Trägermaterialien Materialien umfassen, die aus Saccharose, Maltose-Dextrin, Zitronensäure und Kombinationen hievon ausgewählt sind.

9. Psyllium enthaltende Produkte nach einem der Ansprüche 5 bis 8, in einer zum Mischen in einer Flüssigkeit geeigneten Pulverform, bestehend aus:
(a) 10 % bis 60 % Psylliumschalen;
(b) 35 % bis 90 % Zucker; und
(c) 0 % bis 5 % Maltose-Dextrin.

10. Psyllium enthaltende Produkte nach einem der Ansprüche 5 bis 8, in einer zum Mischen in einer Flüssigkeit geeigneten Pulverform, bestehend aus:
(a) 40 % bis 98 % Psylliumschalen; und
(b) 1 % bis 60 % Maltose-Dextrin.

## Revendications

1. Cosse ou enveloppe de psyllium ,consistant en des particules dont la distribution des tailles est la suivante : moins de 15% ont plus de 177 micromètres (tamis n^{o} 80),au moins 45 % se situent entre 177 et 74 micromètres ( tamis n^{o} 80 à n^{o} 200) et moins de 40 % ont moins de 74 micromètres (tamis n^{o} 200 ) .

2. Cosse de psyllium selon la revendication 1 ,consistant en des particules dont la distribution des tailles est la suivante : moins de 5% ont plus de 250 micromètres (tamis n^{o}60) moins de 10 % ayant plus de 177 micromètres (tamis n^{o} 80),au moins 65 % se situent entre 177 et 74 micromètres ( tamis n^{o}80 à 200),et moins de 25 % ont moins de 74 micromètres (tamis n^{o} 200 ) .

3. Cosse de psyllium selon la revendication 2 ,consistant en des particules dont la distribution des tailles est la suivante : pas de particules ayant plus de 250 micromètres (tamis n^{o} 60) , moins de 5% de particules ayant plus de 177 micromètres ( tamis n^{o} 80) , au moins 75 % se situant entre 177 micromètres et 74 micromètres (tamis n^{o} 80 à 200) ,et moins de 20 % ont moins de 74 micromètres (tamis n^{o} 200 ) .

4. Cosse de psyllium selon la revendication 3 , consistant en des particules dont la distribution des tailles est la suivante : pas de particules ayant plus de 177 micromètres (tamis n^{o} 80 ) ,au moins 80 % se situant entre 177 et 74 micromètres (tamis n^{o} 80 à 200) , et moins de 20 % ont moins de 74 micromètres (tamis N^{o} 200).

5. Produits contenant du psyllium pour administration orale , comprenant :
(a) de la cosse de psyllium ayant une distribution des tailles particulaires selon l'une quelconque des revendica tions 1 à 4 ; et
(b) des matières constituant un support ou un excipient et convenant pour une administration orale à un être humain .

6. Produits contenant du psyllium selon la revendication 5 , sous forme de poudre convenant pour du mélangeage avec un liquide afin de former une boisson contenant du psyllium.

7. Produits contenant du psyllium selon les revendications 5 ou 6,et comprenant en outre des agglomérats de psyllium.

8. Produits contenant du psyllium selon l'une quelconque des revendications 5 à 7,dans lesquels lesdites matières constituant un excipient ou un support comprennent des matières choisies parmi le saccharose ,de la maltodextrine , de l'acide citrique et leurs combinaisons .

9. Produits contenant du psyllium sous forme d'une poudre convenant pour son mélangeage par incorporation à un liquide ,selon l'une quelconque des revendications 5 à 8,et consistant en :
(a) de 10 % à 60 % de cosse ou enveloppe de psyllium;
(b) de 35 % à 90 % de sucre ; et
(c) de 0 % à 5 % de maltodextrine .

10. Produits contenant du psyllium,sous forme de poudre convenant pour son mélangeage par incorporation à un liquide, selon l'une quelconque des revendications 5 à 8 , et consistant en :
(a) de 40 % à 98 % de cosse de psyllium ; et
(b) de 1 % à 60 % de maltodextrine .
